(19) Europäisches Patentamt
European Patent Office
Office européen des brevets

(11) Veröffentlichungsnummer : **0 410 315 B1**

(12) **EUROPÄISCHE PATENTSCHRIFT**

(45) Veröffentlichungstag der Patentschrift :
**29.09.93 Patentblatt 93/39**

(51) Int. Cl.$^5$ : **C07C 227/02,** C07C 229/74

(21) Anmeldenummer : **90113917.0**

(22) Anmeldetag : **20.07.90**

# (54) Verfahren zur Herstellung von 1-Amino-2-carboxyanthrachinonen.

(30) Priorität : **28.07.89 DE 3925060**

(43) Veröffentlichungstag der Anmeldung :
**30.01.91 Patentblatt 91/05**

(45) Bekanntmachung des Hinweises auf die Patenterteilung :
**29.09.93 Patentblatt 93/39**

(84) Benannte Vertragsstaaten :
**CH DE FR GB LI**

(56) Entgegenhaltungen :
**DE-A- 2 933 531**

(73) Patentinhaber : **BASF Aktiengesellschaft
Carl-Bosch-Strasse 38
D-67063 Ludwigshafen (DE)**

(72) Erfinder : **Helwig, Reinhard, Dr.
Theodor-Storm-Strasse 27
D-6718 Gruenstadt (DE)**
Erfinder : **Hoch, Helmut, Dr.
Am Wingertsberg 14
D-6719 Weisenheim (DE)**

EP 0 410 315 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 1-Amino-2-carboxyanthrachinonen der allgemeinen Formel I

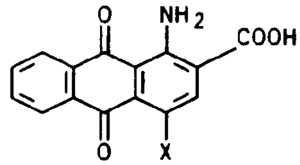

I

in der X Wasserstoff, Chlor oder Brom bedeutet, durch Oxidation eines in 2-Stellung mit einem oxidierbaren C-organischen Rest substituierten 1-Aminoanthrachinons unter alkalischen Bedingungen und anschließendes Ansäuern des Reaktionsgemisches.

1-Amino-2-carboxyanthrachinone und 1-Amino-2-carboxy-4-halogen-anthrachinone sind wichtige Zwischenprodukte für die Herstellung von Dispersions- und Küpenfarbstoffen sowie von Pigmenten. Sie sind bekanntermaßen auf verschiedene Weise zugänglich und zwar meistens durch Oxidation der entsprechenden Anthrachinone, die in 2-Stellung einen oxidierbaren C-organischen Rest tragen, unter alkalischen Bedingungen.

So erhält man die Anthrachinonderivate I durch Oxidation der entsprechenden 2-Hydroxymethylverbindungen mit Oxidationsmitteln wie Kaliumpermanganat (DE-A 21 30 699), durch Oxidation der entsprechenden 2-Methylanthrachinone mit Sauerstoff unter Druck (DE-A 4 99 994) oder der 2-Methyl-, 2-Hydroxymethyl-, 2-Formyl- oder N-substituierten 2-Aminomethylanthrachinone mit Sauerstoff unter stark basischen Bedingungen in mit Wasser mischbaren, polaren aprotischen Lösungsmitteln (DE-A 26 22 227). Ferner ist die Oxidation der 2-Acetylgruppe zur Carboxygruppe mit Hypochloriten bekannt (DE-A 29 33 531).

In saurem Medium ist insbesondere die Oxidation von 1-Nitro-2-methylanthrachinon mit Chrom(VI)verbindungen von Bedeutung (BIOS 987, 13).

Diese Verfahren vermögen jedoch in verschiedener Hinsicht nicht zu befriedigen, sei es aufgrund unzureichender Ausbeuten (das gilt beispielsweise für die Oxidation mit Hypochlorit), des Bedarfs an organischen Lösungsmitteln, des technischen Aufwands durch das Arbeiten unter Druck oder der Schwermetallbelastung des Abwassers.

Der Erfindung lag daher die Aufgabe zugrunde, diesen Mängeln abzuhelfen.

Demgemäß wurde ein neues Verfahren zur Herstellung von 1-Amino-2-carboxyanthrachinonen der allgemeinen Formel I

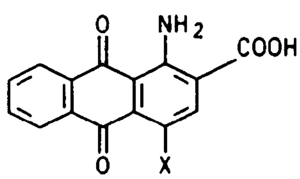

I

in der X Wasserstoff, Chlor oder Brom bedeutet, durch Oxidation eines in 2-Stellung mit einem oxidierbaren C-organischen Rest substituierten 1-Aminoanthrachinons unter alkalischen Bedingungen und anschließendes Ansäuern des Reaktionsgemisches gefunden, welches dadurch gekennzeichnet ist, daß man hierbei ein 1-Amino-2-acetylanthrachinon der allgemeinen Formel II

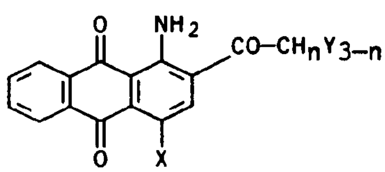

II

in der Y Chlor oder Brom bedeutet und n für 0 bis 2 steht, mit einer peroxidischen Verbindung oder mit Sauerstoff als Oxidationsmittel umsetzt.

Die 1-Amino-2-acetylhalogenanthrachinone II, wie sie als Ausgangsstoffe für das erfindungsgemäße Verfahren eingesetzt werden, sind bekannt und beispielsweise nach der DE-C 11 27 911 durch Halogenierung von 1-Amino-2-acetylanthrachinon, das bereits kernhalogeniert sein kann, in konzentrierter Schwefelsäure oder Eisessig zugänglich.

Allgemein bevorzugte Ausgangsstoffe II sind die 1-Amino-2-dihalogenacetylanthrachinone (n = 1), wobei den in 4-Stellung unsubstituierten Verbindungen (X = H) im Hinblick auf das besonders wichtige 1-Amino-2-carboxyanthrachinon die größte Bedeutung zukommt.

Bevorzugte Oxidationsmittel sind

- Wasserstoffperoxid in Form von 20 bis 40 gew.-%igen wäßrigen Lösungen sowie
- Natriumperoxid.

Daneben eignen sich

- Ammonium- oder Alkalimetallsalze anorganischer Peroxosäuren, wie Percarbonate, vorzugsweise Natriumpercarbonat, Perborate, vorzugsweise Natriumperborat, Peroxodisulfate, vorzugsweise Natriumperoxodisulfat,
- organische Peroxosäuren, wie Peressigsäure oder Perbenzoesäure,
- Sauerstoff oder Sauerstoff enthaltende Gase wie Luft oder
- Gemische dieser Mittel.

Vorzugsweise setzt man die Oxidationsmittel in molarem Überschuß über das Anthrachinonderivat II ein, wobei dieser Überschuß in allgemeinen 4 bis 8 mol, besonders 6 bis 7 mol, beträgt.

Als Basen kommen Alkalimetall- oder Erdalkalimetallhydroxide oder Alkalimetall- oder Erdalkalimetallsalze schwacher Säuren, insbesondere Carbonate, zum Einsatz. Besonders geeignet sind Natrium- oder Kaliumhydroxid.

Die Menge an zugesetzter Base liegt, bezogen auf das Anthrachinonderivat II, im allgemeinen bei 100 bis 250 Gew.-%, vorzugsweise bei 150 bis 200 Gew.-%. Der Basenzusatz kann bei Verwendung eines alkalisch reagierenden Oxidationsmittels entfallen.

Das erfindungsgemäße Verfahren wird vorzugsweise in wäßriger Lösung durchgeführt, jedoch können auch wäßrige Lösungen organischer Lösungsmittel, wie z.B. Dimethylformamid, Dimethylacetamid oder Dimethylsulfoxid, verwendet werden.

Die Reaktionstemperatur beträgt in der Regel 20 bis 100°C, bevorzugt 30 bis 70°C.

Verfahrenstechnisch geht man im allgemeinen so vor, daß man das Anthrachinonderivat II in wäßriger bzw. alkalischer Suspension vorlegt und das Oxidationsmittel mit oder ohne Basenzusatz bei der Reaktionstemperatur zugibt. Es ist aber auch möglich, das Oxidationsmittel vorzulegen.

Wird ein gasförmiges Oxidationsmittel, beispielsweise Luft, verwendet, so wird dieses vorzugsweise bei Atmosphärendruck durch die Reaktionslösung geleitet. Das Ende der Umsetzung kann dünnschichtchromatographisch bestimmt werden.

Da Reaktion und Neutralisation im allgemeinen unter Gasentwicklung ablaufen (Sauerstoff oder Kohlendioxid), kann sich der Zusatz geringer Mengen von Schaumdämpfungsmitteln, wie beispielsweise den Natriumsalzen von Paraffinsulfonsäuren, empfehlen.

Die Aufarbeitung des Reaktionsgemisches nimmt man wie üblich vor, und zwar vorzugsweise indem man die Verfahrensprodukte I durch Ansäuern ausfällt, abtrennt und wäscht.

Man erhält die Anthrachinone I in bereits so hoher Reinheit, daß sie unmittelbar zu weiteren Zwecken wie vor allem der Farbstoffsynthese eingesetzt werden können. Die Reinheitsüberprüfung erfolgt wie üblich chromatographisch.

Das erfindungsgemäße Verfahren zeichnet sich gegenüber den bisher bekannten Verfahren durch hohe Ausbeuten und hohe Reinheiten der 1-Amino-2-carboxyanthrachinone I sowie durch verfahrenstechnische Einfachheit aus.

Eine besonders bevorzugte Anwendung dieses Verfahrens besteht in der Herstellung des unsubstituierten 1-Amino-2-carboxyanthrachinons, das für zahlreiche Farbstoffsynthesen im Bereich der Küpen- und Dispersionsfarbstoffe ein wichtiges Zwischenprodukt darstellt.

Beispiele

Herstellung von 1-Amino-2-carboxyanthrachinonen der Formel

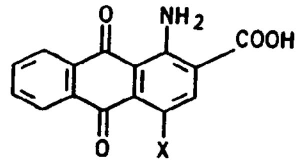

(X: Wasserstoff, Chlor, Brom)

Eine Suspension aus a mol eines 1-Aminoanthrachinons II in b g Wasser wurde bei 55 bis 60°C innerhalb 1 h gleichzeitig mit c g 50 gew.-%iger Natronlauge und d g eines Oxidationsmittels versetzt und 2 h bei 60°C gerührt.

Dann wurde unter Konstanthalten der Temperatur solange 75 gew.-%ige Schwefelsäure zugegeben, bis ein pH-Wert ≦2 erreicht war. Das auf diese Weise ausgefällte 1-Amino-2-carboxyanthrachinon wurde abgetrennt, mit Wasser gewaschen und anschließend getrocknet.

Die Einzelheiten dieser Versuche sowie deren Ergebnisse sind der nachstehenden Tabelle zu entnehmen.

Tabelle

| Beispiel | a mol | 1-Amino-anthrachinon II | b g Wasser | c g 50 gew.-%ige Natronlauge | d g | Oxidations-mittel | Ausbeute | Gehalt an I | absolute Ausbeute |
|---|---|---|---|---|---|---|---|---|---|
| 1 | 0,36 | -2-dichloracetyl- (Smp. 221-3°C) | 1200 | 444 | 264 | Perhydrol* | 93 g | 93 % | 90 % |
| 2 | 0,09 | -2-dichloracetyl- | 375 | - | 40 | Natriumperoxid | 22 g | 96 % | 88 % |
| 3 | 0,09 | -2-dichloracetyl- | 300 | 111 | 2,5 + 60 | Natriumperoxid Perhydrol* | 22 g | 94 % | 86 % |
| 4 | 0,09 | -2-dichloracetyl- | 300 | 111 | 61 | Natriumper-carbonat | 22 g | 93 % | 85 % |
| 5 | 0,09 | -2-dichloracetyl- | 300 | 111 | 100 | Natriumper-borat | 22 g | 93 % | 85 % |
| 6 | 0,36 | -2-dibromacetyl- (Smp. 210-3°C) | 1200 | 444 | 264 | Perhydrol* | 93 g | 93 % | 90 % |
| 7 | 0,09 | -2-dibromacetyl- | 375 | - | 40 | Natriumperoxid | 22 g | 96 % | 88 % |
| 8 | 0,09 | -2-dibromacetyl- | 300 | 111 | 2,5 + 60 | Natriumperoxid Perhydrol* | 22 g | 94 % | 86 % |

Tabelle (Fortsetzung)

| Beispiel | a mol | 1-Amino-anthrachinon II | b g Wasser | c g 50 gew.-%ige Natronlauge | d g | Oxidations-mittel | Ausbeute | Gehalt an I | absolute Ausbeute |
|---|---|---|---|---|---|---|---|---|---|
| 9 | 0,36 | -2-dichloracetyl-4-chlor- (Smp. 204-6°C) | 1200 | 444 | 264 | Perhydrol* | 99 g | 93 % | 85 % |
| 10 | 0,36 | -2-dibromacetyl-4-chlor- (Smp. 235-6°C) | 1200 | 444 | 264 | Perhydrol* | 100 g | 95 % | 88 % |
| 11 | 0,36 | -2-dichloracetyl-4-brom- (Smp. 208-11°C) | 1200 | 444 | 264 | Perhydrol* | 114 g | 92 % | 84 % |
| 12 | 0,36 | -2-dibromacetyl-4-brom- (Smp. 231-4°C) | 1200 | 444 | 264 | Perhydrol* | 114 g | 92 % | 84 % |

(*: Perhydrol ≙ 30 gew.-%iges Wasserstoffperoxid)

## Patentansprüche

1. Verfahren zur Herstellung von 1-Amino-2-carboxyanthrachinonen der allgemeinen Formel I

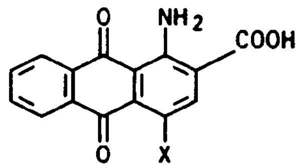

I

in der X Wasserstoff, Chlor oder Brom bedeutet, durch Oxidation eines in 2-Stellung mit einem oxidierbaren C-organischen Rest substituierten 1-Aminoanthrachinons unter alkalischen Bedingungen und anschließendes Ansäuern des Reaktionsgemisches, dadurch gekennzeichnet, daß man hierbei ein 1-Amino-2-acetylanthrachinon der allgemeinen Formel II

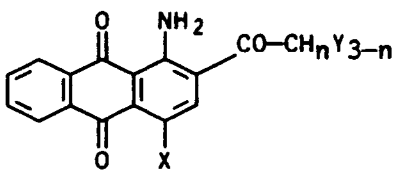

II

in der Y Chlor oder Brom bedeutet und n für 0 bis 2 steht, mit einer peroxidischen Verbindung oder mit Sauerstoff als Oxidationsmittel umsetzt.

2. Verfahren nach Anspruch 1, dadurch gekennzeichnet, daß man ein 1-Amino-2-acetylanthrachinon II einsetzt, in welchem n für 1 steht.

3. Verfahren nach den Ansprüchen 1 oder 2, dadurch gekennzeichnet, daß man ein 1-Amino-2-acetylanthrachinon II einsetzt, in welchem X Wasserstoff bedeutet.

4. Verfahren nach den Ansprüchen 1 bis 3, dadurch gekennzeichnet, daß man als Oxidationsmittel Ammonium- oder Alkalimetallpercarbonate, -perborate oder -peroxodisulfate, Wasserstoffperoxid, Alkalimetallperoxide, Peressigsäure oder Perbenzoesäure verwendet.

5. Verfahren nach den Ansprüchen 1 bis 4, dadurch gekennzeichnet, daß man die Umsetzung in wäßriger Lösung durchführt.

6. Verfahren nach den Ansprüchen 1 bis 5, dadurch gekennzeichnet, daß man die Umsetzung bei 30 bis 70°C durchführt.

## Claims

1. A process for preparing 1-amino-2-carboxyanthraquinones of the general formula I

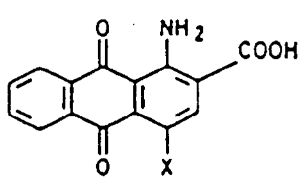

I

where X is hydrogen, chlorine or bromine, by oxidation under alkaline conditions of a 1-aminoanthraquinone substituted in the 2-position by an oxidizable organocarbon radical and subsequent acidification of the reaction mixture, which comprises reacting a 1-amino-2-acetylanthraquinone of the general formula

(II)

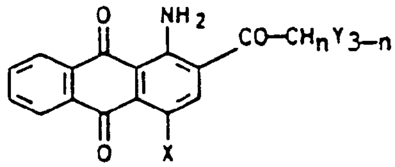

II

where Y is chlorine or bromine and n is from 0 to 2, with a peroxidic compound or with oxygen as oxidizing agent.

2. A process as claimed in claim 1, wherein the 1-amino-2-acetylanthraquinone II used has n = 1.

3. A process as claimed in claim 1 or 2, wherein the 1-amino-2-acetylanthraquinone II used has X = hydrogen.

4. A process as claimed in any of claims 1 to 3, wherein the oxidizing agent used is an ammonium or alkali metal percarbonate, perborate or peroxodisulfate, hydrogen peroxide, an alkali metal peroxide, peracetic acid or perbenzoic acid.

5. A process as claimed in any of claims 1 to 4, wherein the reaction is carried out in aqueous solution.

6. A process as claimed in any of claims 1 to 5, wherein the reaction is carried out at from 30 to 70°C.

**Revendications**

1. Procédé de préparation de 1-amino-2-carboxyanthraqulnones de formule générale

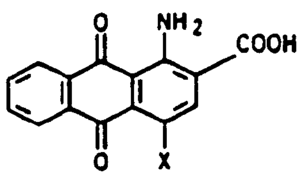

I

dans laquelle X représente un atome d'hydrogène, de chlore ou de brome, par oxydation, dans des conditions alcalines, d'une 1-aminoanthraquinone substituée en position 2 par un reste C-organique oxydable, suivie d'acidification du mélange réactionnel, caractérisé en ce qu'on fait réagir une 1-amino-2-acétylanthraquinone de formule générale II

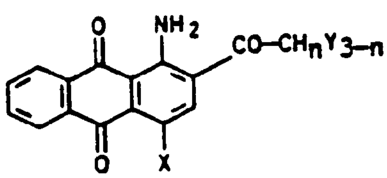

II

dans laquelle Y représente un atome de chlore ou de brome et n est mis pour 0 à 2, avec un composé de type peroxyde ou avec de l'oxygène en tant qu'oxydant.

2. Procédé selon la revendication 1, caractérisé en ce qu'on utilise une 1-amino-2-acétylanthraquinone II dans laquelle n est mis pour 1.

3. Procédé selon la revendication 1 ou 2, caractérisé en ce qu'on utilise une 1-amino-2-acétylanthraquinone

II dans laquelle X représente un atome d'hydrogène.

4. Procédé selon l'une quelconque des revendications 1 à 3, caractérisé en ce qu'on utilise, comme oxydant, des percarbonates, des perborates ou des peroxodisulfates d'ammonium ou de métaux alcalins, du peroxyde d'hydrogène, des peroxydes de métaux alcalins, de l'acide peracétique ou de l'acide perbenzoïque.

5. Procédé selon l'une quelconque des revendications 1 à 4, caractérisé en ce qu'on conduit la réaction en solution aqueuse.

6. Procédé selon l'une quelconque des revendications 1 à 5, caractérisé en ce qu'on conduit la réaction à une température de 30 à 70°C.